# EUROPEAN PATENT APPLICATION

(11) **EP 1 868 275 A2**
(43) Date of publication of application: **19.12.2007**
(21) Application number: 07252395.4
(22) Date of filing: 13.06.2007
(51) Int. Cl.: H02J 7/00

(54) **Inductive charging of tools on surgical tray**

(30) Priority: 14.06.2006 US 424089
(71) Applicant: Biosense Webster, Inc., Diamond Bar, CA 91765 (US)
(72) Inventor: Govari, Assaf, 34400 Haifa (IL); Altmann, Andres Claudio, 34614 Haifa (IL); Ephrath, Yaron, 37501 Karkur (IL)
(74) Representative: Tunstall, Christopher Stephen

(57) **Abstract**

Electrical apparatus includes a tray (20), having a surface (28) on which a tool containing a rechargeable power source can be placed, and a magnetic field generator (30), which is located below the surface of the tray and is operative to generate a time-varying magnetic field of sufficient power at the surface of the tray so as to inductively charge the power source in the tool.

## Description

### FIELD OF THE INVENTION

The present invention relates generally to cordless electrical tools, and specifically to methods and devices for inductive recharging of electrical tools used in medical procedures.

### BACKGROUND OF THE INVENTION

Electrical surgical tools are commonly used in operating rooms. For example, electrocautery devices are used in many procedures, and orthopedists commonly use power saws and drills. Operating rooms are often crowded with personnel and equipment, and electrical cords for powered surgical tools add to the difficulty of maneuvering and performing the operation. Maintaining the sterility of power cords can also be problematic. In response to these difficulties, cordless, battery-powered surgical tools, such as cordless orthopedic drills and saws, are becoming increasingly popular.

In the course of a surgical procedure, the battery in a surgical tool may run down. In this case, the battery must be recharged or replaced while maintaining sterile conditions. The tool, and possibly the battery and charger (if used), must generally be capable of withstanding sterilization. U.S. Patent 4,288,733, for example, describes a battery charger system suited for use in a sterilized environment, such as an operating room. The system includes a sterilizable battery pack adapted for connection to a non-sterilized charger through a sterilizable tray-interface. The tray-interface includes a connecting structure on an upper side for mechanical and electrical connection to the battery pack and additional connecting structure on a lower side for mechanical and electrical connection to the charger. A sterilizable drape is constrained between the tray-interface and the charger to isolate the charger from the sterilized environment.

### SUMMARY OF THE INVENTION

Embodiments of the present invention provide a charging tray for inductive charging of cordless electrical tools that simplifies recharging, while maintaining sterility of the tools. The tray may have the form of tool trays that are commonly used in the operating room. A magnetic field generator is located below the surface of the tray and generates a time-varying magnetic field of sufficient power at the surface of the tray so as to inductively charge tools that are placed on the tray. Inductive charging is advantageous in this context since it does not require that there be any physical contact between the field generator and the tools. The tools are recharged whenever they are placed on the tray, thus reducing the chances that a tool will run out of power in the course of a procedure.

Some embodiments of the present invention are arranged so that tools may be placed on the tray in arbitrary orientations, without special receptacles for holding the tools in some predetermined position during charging. Typically, for this purpose, the inductive charging circuit in the tools is configured to receive power from the magnetic field produced by the field generator regardless of the orientation of the tool. This feature may be achieved, for example, by incorporating in the charging circuit two or more receiver coils with different orientations. In such embodiments, the tray may also be covered with a sterile drape without affecting its function.

There is therefore provided, in accordance with an embodiment of the present invention, electrical apparatus, including:
a tray, having a surface on which a tool containing a rechargeable power source can be placed; and
a magnetic field generator, which is located below the surface of the tray and is operative to generate a time-varying magnetic field of sufficient power at the surface of the tray so as to inductively charge the power source in the tool.

The surface of the tray may be flat. Alternatively, the surface of the tray may have one or more recesses for receiving the tool.

Typically, the magnetic field generator includes a coil, which is encapsulated in the tray.

In one embodiment, the apparatus includes a cover, which is configured to be placed over the tool on the tray so as to confine the magnetic field to a vicinity of the tray. Additionally or alternatively, the apparatus may include a sterile drape, which is placed over the surface of the tray, wherein the tool is charged by the magnetic field generator while the tool lies on the sterile drape.

There is also provided, in accordance with an embodiment of the present invention, electrical apparatus, including:
a cordless electric-powered tool, including a rechargeable power source and a charging circuit for charging the power source;
a tray, having a surface on which the tool can be placed; and
a magnetic field generator, which is located below the surface of the tray and is operative to generate a time-varying magnetic field of sufficient power at the surface of the tray so as to inductively charge the power source in the tool via the charging circuit.

In some embodiments, the magnetic field generator, tray and charging circuit are configured so that the magnetic field generator charges the power source in any orientation in which the tool is placed on the surface. Typically, the charging circuit includes a plurality of coils, which have different, respective axes and are adapted to inductively receive energy from the time-varying magnetic field. Additionally or alternatively, the magnetic field generator and tray are configured so that the magnetic field generator charges the power source in the tool at any location at which the tool is placed on the surface.

There is additionally provided, in accordance with an embodiment of the present invention, a method for recharging a tool containing a rechargeable power source, the method including:
placing the tool on a surface of a tray; and
generating a time-varying magnetic field, using a magnetic field generator located below the surface of the tray of sufficient power at the surface of the tray so as to inductively charge the power source in the tool.

The present invention will be more fully understood from the following detailed description of the embodiments thereof, taken together with the drawings in which:

### BRIEF DESCRIPTION OF THE DRAWINGS

Fig. 1 is a schematic, pictorial illustration showing a tool tray with inductive charging capability in use during a surgical procedure, in accordance with an embodiment of the present invention;
Fig. 2 is a schematic bottom view of a tool tray with inductive charging coils, in accordance with an embodiment of the present invention;
Fig. 3 is a schematic side view of a tool tray with a rechargeable tool placed thereon, in accordance with an embodiment of the present invention; and
Fig. 4 is a schematic cutaway view of a rechargeable tool, in accordance with an embodiment of the present invention.

### DETAILED DESCRIPTION OF EMBODIMENTS

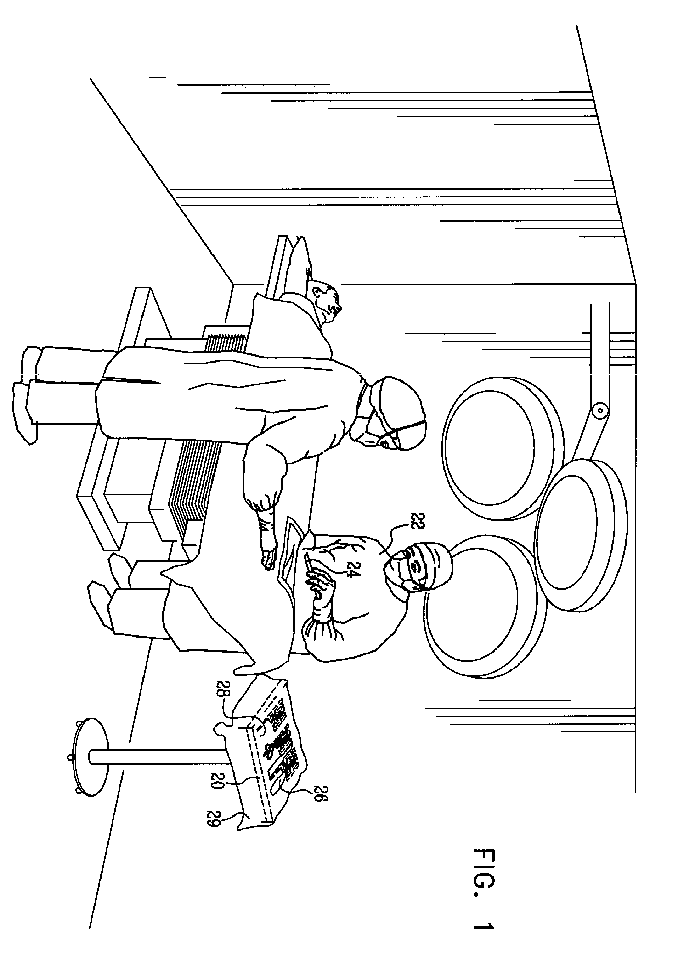

Fig. 1 is a schematic, pictorial illustration showing a tool tray 20 with inductive charging capability in use during a surgical procedure, in accordance with an embodiment of the present invention. A surgeon 22 uses cordless, electrically-powered tools 24, 26 during the procedure. When a given tool is not in use, the surgeon places it on a surface 28 of tool tray 20. While the tool is on the tray, it is inductively charged by a magnetic field produced by a field generator below the surface of the tray (shown in the figures that follow).

Tool tray 20 and tools 24, 26 may be designed, as described hereinbelow, so that the tools are charged regardless of the orientation in which they are placed on the tray. The charging magnetic field may be concentrated in one area of the tray, or it may be generated over the entire tray surface, so that the tool is charged regardless of location on the tray, as well. Thus, there is no need for receptacles to hold the tools on the tray. Surface 28 may be flat, and the operating room staff may use the tray to hold tools in the same manner as conventional tool trays without charging capability. The tray may be covered with a sterile drape 29 without compromising its function. Alternatively, tray 20 may be constructed so as to permit the tray to be sterilized between uses, in which case drape 29 may not be needed. (Tools 24 and 26 are typically constructed so as to permit them to be sterilized by methods known in the art, such as autoclaving or chemical sterilization.)

In alternative embodiments of the present invention (not shown in the figures), the tool tray may have one or more recesses in its upper surface in which tools are placed for recharging. In such embodiments, the charging magnetic field may be localized in the area of the recesses, and the tools may be constrained by the shape of the recesses to an orientation that maximizes energy transfer from the magnetic field generator to the tool. Alternatively, the optimal locations and orientations of the tools on the tray may simply be marked graphically on the tray surface or drape, so as to indicate to surgeon 22 how and where to place the tools on the tray. Additionally or alternatively, the tools themselves may be marked and/or shaped to indicate the proper orientation.

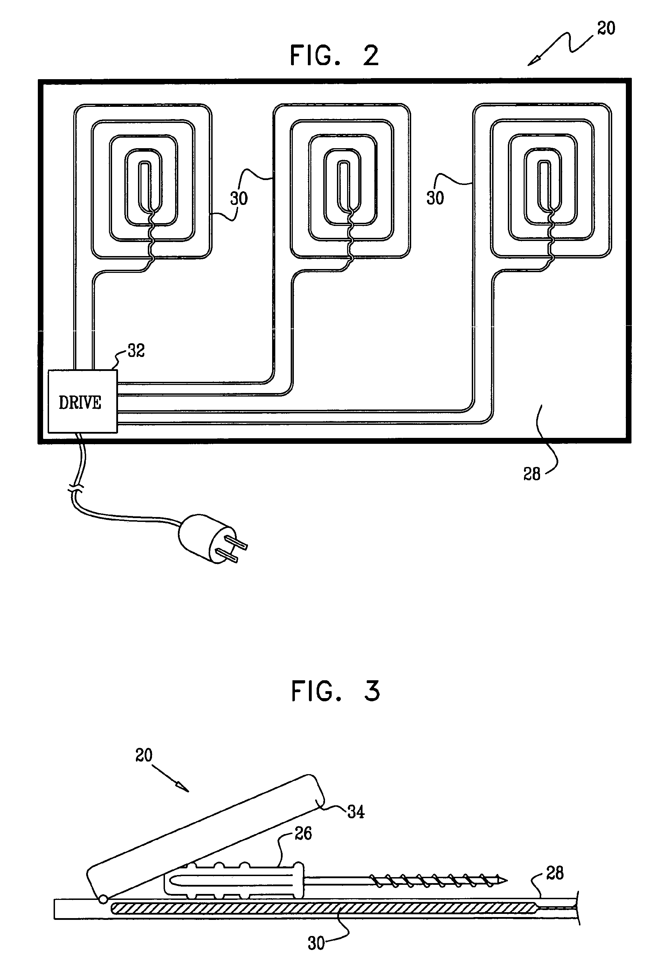

Fig. 2 is a schematic bottom view of tool tray 20, in accordance with an embodiment of the present invention. One or more magnetic field generators 30 are positioned below surface 28 of the tray. In the pictured embodiment, the field generators have the form of flat coils, which may be encapsulated within the tray. Alternatively, the field generators may be positioned below the tray. Tray 20 is typically made of a plastic or other non-magnetic material, so as not to block the passage of the magnetic fields from the field generators below surface 28 to tools that are placed on the surface.

Although the field generators shown in Fig. 2 comprise a certain type of coils, any suitable field generator design may be used in tray 20, such as coils of other shapes and forms, with or without a magnetic core, or a "bird cage" magnetic field generator, for example. The field generator or generators may be configured to generate the magnetic field over all of surface 28, or they may alternatively be positioned and configured to generate the field only in a certain area or areas of the surface. The field generators are typically designed so that the field they generate is localized near the surface of the tray, thereby minimizing interference by the magnetic field with sensors and other equipment used in the operating room. Shielding may be added to the tray (including an optional tray cover) to limit the interference with other equipment, as well as for safety reasons.

A driver circuit 32 drives an alternating current through field generators 30 in order to generate magnetic fields at the desired frequency. Typically, the field frequency can be anywhere in the range of 100 kHz - 30 MHz, depending on the application and the resonant frequency of the charging circuit in tools 24, 26 (as described further hereinbelow). Alternatively, higher or lower magnetic field frequencies may be used.

Fig. 3 is a schematic side view of tool tray 20 with tool 26 resting on surface 28, in accordance with an embodiment of the present invention. Field generator 30 generates a magnetic field with field lines passing vertically through surface 28 and intercepting tool 26. Optionally, a magnetic shield 34 may be lowered over tool 26 while it is being charged by field generator 30. This shield typically comprises a metal or other material with high magnetic permeability, in order to confine the radiation of the magnetic field to the area of tray 20.

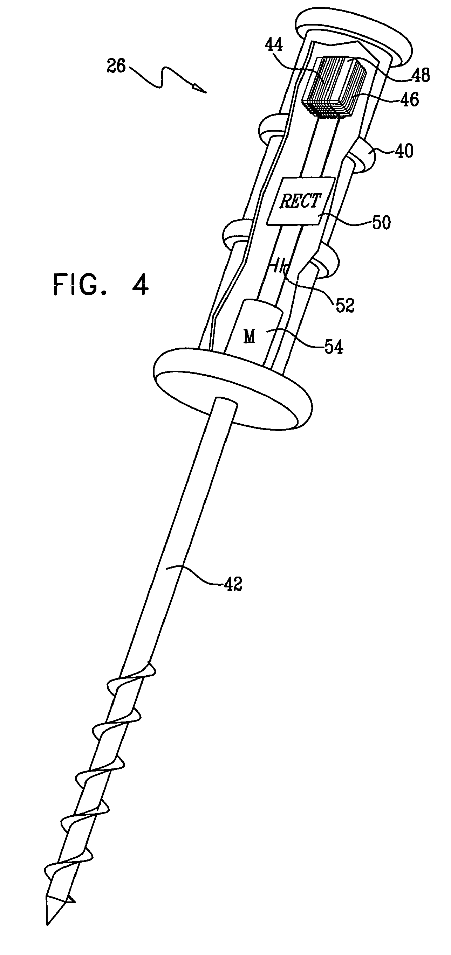

Fig. 4 is a schematic, cutaway view of tool 26, showing circuitry contained in a handle 40 of the tool, in accordance with an embodiment of the present invention. Coils 44 and 46 are wound on a core 48, which typically comprises a ferrite or other magnetic material. In this embodiment, coils 44 and 46 have mutually-orthogonal axes. As a result, when tool 26 is laid on tray 20, at least one of coils 44 and 46 will be suitably oriented to receive energy from the magnetic field perpendicular to surface 28 that is produced by field generators 30, regardless of the orientation of the tool. ("Orientation" in this sense can mean either the direction in which the tool is pointing or the roll angle of the tool about its longitudinal axis, or both.) For optimal energy transfer, coils 44 and 46 are typically connected in respective resonant circuits, whose resonant frequency matches the driving frequency of field generators 30.

Coils 44 and 46 are connected to a rectifier circuit 50, which provides a DC input to charge a power source 52. The rectifier circuit may also comprise a regulator and power management controller, as are known in the art, in order to prevent overcharging of the power source. Power source 52 may comprise a super-capacitor or a rechargeable battery, of any suitable type known in the art. The power source in this embodiment supplies energy to a motor 54, which drives a shaft 42. Alternatively, the power source may be coupled to drive any other suitable type of mechanism or device, such as a saw, drill, electrocautery scalpel, ablation head, laser or other light source, as well as other electrical circuit components, such as amplifiers, microcontrollers and wireless communication electronics.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. Electrical apparatus, comprising:
a tray, having a surface on which a tool containing a rechargeable power source can be placed; and
a magnetic field generator, which is located below the surface of the tray and is operative to generate a time-varying magnetic field of sufficient power at the surface of the tray so as to inductively charge the power source in the tool.

2. The apparatus according to claim 1, wherein the magnetic field generator and tray are configured so that the magnetic field generator charges the power source in the tool in any orientation in which the tool is placed on the surface.

3. The apparatus according to claim 1, wherein the magnetic field generator and tray are configured so that the magnetic field generator charges the power source in the tool at any location at which the tool is placed on the surface.

4. The apparatus according to claim 1, wherein the surface of the tray is flat.

5. The apparatus according to claim 1, wherein the surface of the tray has one or more recesses for receiving the tool.

6. The apparatus according to claim 1, wherein the magnetic field generator comprises a coil, which is encapsulated in the tray.

7. The apparatus according to claim 1, and comprising a cover, which is configured to be placed over the tool on the tray so as to confine the magnetic field to a vicinity of the tray.

8. The apparatus according to claim 1, and comprising a sterile drape, which is placed over the surface of the tray, wherein the tool is charged by the magnetic field generator while the tool lies on the sterile drape.

9. Electrical apparatus, comprising:
a cordless electric-powered tool, comprising a rechargeable power source and a charging circuit for charging the power source;
a tray, having a surface on which the tool can be placed; and
a magnetic field generator, which is located below the surface of the tray and is operative to generate a time-varying magnetic field of sufficient power at the surface of the tray so as to inductively charge the power source in the tool via the charging circuit.

10. The apparatus according to claim 9, wherein the magnetic field generator, tray and charging circuit are configured so that the magnetic field generator charges the power source in any orientation in which the tool is placed on the surface.

11. The apparatus according to claim 10, wherein the charging circuit comprises a plurality of coils, which have different, respective axes and are adapted to inductively receive energy from the time-varying magnetic field.

12. The apparatus according to claim 9, wherein the magnetic field generator and tray are configured so that the magnetic field generator charges the power source in the tool at any location at which the tool is placed on the surface.

13. The apparatus according to claim 9, wherein the surface of the tray is flat.

14. The apparatus according to claim 9, wherein the surface of the tray has one or more recesses for receiving the tool.

15. The apparatus according to claim 9, wherein the magnetic field generator comprises a coil, which is encapsulated in the tray.

16. The apparatus according to claim 9, and comprising a cover, which is configured to be placed over the tool on the tray so as to confine the magnetic field to a vicinity of the tray.

17. The apparatus according to claim 9, and comprising a sterile drape, which is placed over the surface of the tray, wherein the tool is charged by the magnetic field generator while the tool lies on the sterile drape.

18. A method for recharging a tool containing a rechargeable power source, the method comprising:
placing the tool on a surface of a tray; and
generating a time-varying magnetic field, using a magnetic field generator located below the surface of the tray of sufficient power at the surface of the tray so as to inductively charge the power source in the tool.

19. The method according to claim 18, wherein the magnetic field generator and tray are configured so that the magnetic field generator charges the power source in the tool in any orientation in which the tool is placed on the surface.

20. The method according to claim 18, wherein the magnetic field generator and tray are configured so that the magnetic field generator charges the power source in the tool at any location at which the tool is placed on the surface.

21. The method according to claim 18, wherein the surface of the tray is flat.

22. The method according to claim 18, wherein the surface of the tray has one or more recesses for receiving the tool.

23. The method according to claim 18, and comprising placing a sterile drape over the surface of the tray, wherein placing the tool comprises laying the tool on the sterile drape.
